# EUROPEAN PATENT SPECIFICATION

(11) **EP 1 676 540 B1**
(45) Date of publication and mention of the grant of the patent: **18.08.2010**
(21) Application number: 05257909.1
(22) Date of filing: 21.12.2005
(51) Int. Cl.: A61B 19/00

(54) **Apparatus for performing a voice-assisted orthopaedic surgical procedure**
Gerät, um einen orthopädischen Eingriff sprachgesteuert durchzuführen
Appareil pour chirurgie orthopédique a assistance vocale

(30) Priority: 29.12.2004 US 640155 P; 31.03.2005 US 94955
(43) Date of publication of application: 05.07.2006
(73) Proprietor: DePuy Products, Inc., Warsaw, IN 46581 (US)
(72) Inventor: DiSilvestro, Mark R., Columbia City, IN 46725 (US); Dietz, Terry L., Columbia City, IN 46725 (US); Sherman, Jason T., Warsaw, IN 46582 (US)
(74) Representative: Belcher, Simon James

(56) References cited:
- EP-A- 1 172 064
- WO-A-2004/070580
- US-A- 5 303 148
- US-A1- 2001 016 684

## Description

The present disclosure relates generally to computer assisted surgery systems for use in the performance of orthopaedic procedures.

There is an increasing adoption of minimally invasive orthopaedic procedures. Because such surgical procedures generally restrict the surgeon's ability to see the operative area, surgeons are increasingly relying on computer systems, such as computer assisted orthopaedic surgery (CAOS) systems, to assist in the surgical operation. CAOS systems assist surgeons in the performance of orthopaedic surgical procedures by, for example, displaying images illustrating surgical steps of the surgical procedure being performed. In typical CAOS systems, the surgeon operates or otherwise interacts with the CAOS system via one or more direct interface devices such as a keyboard, mouse, touch screen, and the like.

WO-A-2004/070580 discusses a computer-assisted knee replacement system. The system includes an input device, a display device and a processor. The processor can run application-specific programs to assist a surgeon with planning and/or navigation. This includes displaying predefined images corresponding to steps of a surgical procedure.

EP-A-1172064 discusses a method and apparatus for accessing medical data over a network. It includes a controller, display device and a microphone in one embodiment. Medical data can be captured and stored using the microphone during surgery or examination.

US-5303148 relates to a voice actuated volume image controller and display controller. It includes a microphone, speech processor and display. In response to spoken commands a volume imager generates an image representation which is sent to the display.

The invention provides a system for assisting a surgeon in performing an orthopaedic surgical procedure as defined in appended claim 1. The system includes a display device such as, for example, a display monitor. The system also includes a microphone configured to transmit voice commands received from the surgeon. The microphone may be configured to be worn by the surgeon. The system also includes a controller electrically coupled to the display device. The controller is communicatively coupled to the microphone via, for example, a wired or wireless connection. The contoller may be configured to control the display device to display a number of images of surgical steps of the orthopaedic surgical procedure in response to corresponding voice commands. The controller may form a portion of a CAOS system. The controller may also include a voice recognition device which may be embodied, in part, as a software program. The surgeon may use voice commands to control functions of the system including, for example, view different surgical images, change user preferences of the display monitor, control other peripheral devices connected to the system, receive data from connected peripheral devices, identify surgical tools, record surgical notes, etc.

Accordingly, in one aspect, the invention provides a system for assisting a surgeon in performing an orthopaedic surgical procedure, the system comprising:
a display device,
a microphone configured to transmit voice commands from the surgeon, and
a controller electrically coupled to the display device and communicatively coupled to the microphone, the controller being configured to control the display device to display a number of images of the orthopaedic surgical procedure in response to at least one of the voice commands. The invention may be use in the following methods, which are not part of the invention:

The invention may be used in a method for assisting a surgeon in performing an orthopaedic surgical procedure, the method comprising:
displaying an image of an orthopaedic surgical procedure on a display device,
receiving voice commands from the surgeon via a microphone, and
modifying the image displayed on the display device in response to receipt of at least one of the voice commands.

The invention may also be used in a method of making an electronic record of a completed orthopaedic surgical procedure, the method comprising:
retrieving a number of images of the completed orthopaedic surgical procedure from a storage device;
receiving verbal communication from a surgeon relating to at least one of the number of images; and
storing the verbal communication in association with the number of images.

The invention may also be used in a method for operating a computer assisted orthopaedic surgery system, the method comprising controlling functions of the computer assisted orthopaedic surgery system via voice commands.

The method can include the step of controlling operation of an orthopaedic surgical instrument in response to receipt of at least one of the voice commands. For example, the controlling step can involve activating the orthopaedic surgical instrument.

Preferably, the controller includes a transmitter, and is configured to communicate with an orthopaedic surgical instrument via the transmitter to control functions of the orthopaedic surgical instrument in response to at least one of the voice commands. For example, the controller may be configured to activate the orthopaedic surgical instrument in response to at least one of the voice commands, and/or to de-activate the orthopaedic surgical instrument in response to at least one of the voice commands, and/or to begin recording voice communication of the surgeon in response to at least one of the voice commands, and/or to store the voice communication (especially electronically) in a storage device.

A method not forming part of the invention for assisting a surgeon in performing an orthopaedic surgical procedure is also disclosed. The method may include displaying an image of a surgical step of the orthopaedic surgical procedure on a display device. The method may also include receiving voice commands from the surgeon via a microphone such as a wireless microphone. The method may further include modifying the image displayed on the display device in response to a corresponding one of the voice commands. The image may be modified by, for example, displaying another surgical image such a subsequent surgical step of the procedure, rendering the anatomical result of a proposed procedure, and the like. The method may also include controlling and/or receiving data from orthopaedic surgical instruments in response to corresponding voice commands.

A method not forming part of the invention for operating a computer assisted orthopaedic surgery system is also disclosed. The method may include controlling functions of the computer assisted orthopaedic surgery system via voice commands.

An orthopaedic surgical system is disclosed, which includes an orthopaedic surgical instrument. The system includes a microphone configured to transmit voice commands received from a user of the orthopaedic surgical instrument such as a surgeon. The system further includes a controller communicatively coupled to the microphone and the orthopaedic surgical instrument. The controller may be configured to communicate with the orthopaedic surgical instrument to control functions of the instrument in response to corresponding voice commands. For example, the controller is configured to communicate with the instrument to activate an indicator located on the instrument to identify the instrument.

A system for recording information concerning an orthopaedic surgical procedure while the orthopaedic surgical procedure is being performed by a surgeon is also disclosed. The system may include a display device configured to display images of surgical steps of the orthopaedic surgical procedure. The system may also include a microphone configured to transmit verbal communication received form the surgeon. The verbal communication may include a voice command. The system may further include a controller communicatively coupled to the microphone. The controller may be configured to electronically record a portion of the verbal communication in response to the voice command. The controller may record the portion of the verbal communication in any suitable electronic format. In addition, the controller may be configured to communicate with a printer at the request of the user to generate a text hardcopy of the recorded verbal communication.

Embodiments of the invention will now be described by way of example with reference to the accompanying drawings, in which:
FIG. 1 is a simplified block diagram of a computer assisted orthopaedic surgery system having voice control capability;
FIG. 2 is a simplified block diagram of a system for assisting a surgeon in performing an orthopaedic surgical procedure;
FIG. 3 is a simplified block diagram of the controller of the system of FIG. 2; and
FIG. 4 is a flowchart of an exemplary control algorithm executed by the systems of FIGS. 1-3.

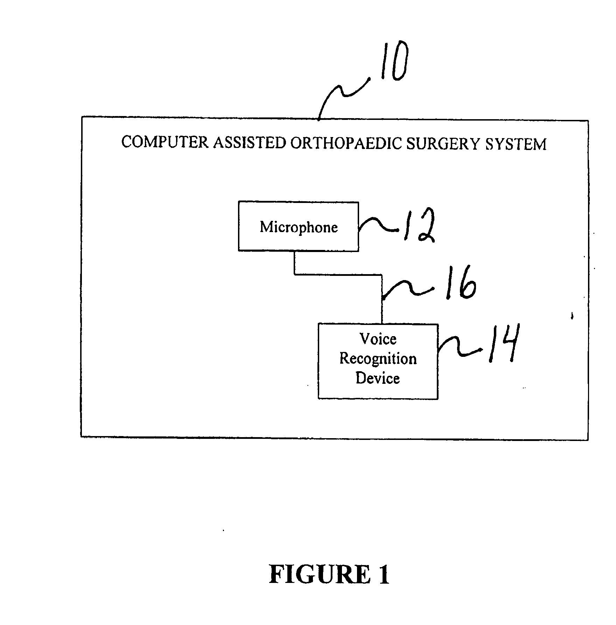

Referring to the drawings, FIG. 1 shows a computer assisted orthopaedic surgery (CAOS) system 10 which includes a microphone 12 and a voice recognition device 14. The CAOS system 10 may be embodied as any type of computer assisted orthopaedic surgery system. Illustratively, the CAOS system 10 can be of the kind which is sold by DePuy Orthopaedics Inc under the trade mark Ci. The microphone 12 is connected to the voice recognition device 14 via a communication link 16. The microphone 12 may be any type of microphone or other receiving device capable of receiving voice commands from an orthopaedic care provider (e.g., the surgeon). The microphone 12 may be wired (i.e., the communication link 16 may be a wired communication link) or wireless (i.e., the communication link 16 is a wireless communication link). The microphone 12 may be attached to a support structure, such as a ceiling or wall of the operating room, so as to be positionable over the surgical area. Alternatively, the microphone 12 may be appropriately sized and configured to be worn, such as on the surgeons head or clothing, or held by the surgeon or other surgical staff member. For example, in some embodiments, the microphone 12 is an ear or throat microphone. Further, the microphone 12 may be incorporated into a unit worn by the surgeon as part of a heads-up display. As such, the term microphone, as used herein, is intended to include any transducer device capable of transducing an audible sound into an electrical signal.

The voice recognition device 14 may be embodied as electrical hardware, software, or a combination thereof. In one embodiment, the voice recognition device 14 is a software program executed by a computer (not shown) of the CAOS system 10. In one particular embodiment, the voice recognition device 14 can use software that is sold by Scansoft of Peabody, Massachusetts under the trade mark Dragon NaturallySpeaking Medical Software (version 8).

The CAOS system 10 may be used by an orthopaedic surgeon to assist in any type of orthopaedic surgical procedure including, for example, a total knee replacement procedure. The CAOS system 10 may be programmed or configured to display images of the individual surgical steps which form the orthopaedic surgical procedure being performed. The images may be graphically rendered images or graphically enhanced photographic images. For example, the images may include three dimensional rendered images of the relevant anatomical portions of a patient. The surgeon may interact with the CAOS system 10 to display the images of the various surgical steps in sequential order. In addition, the surgeon may interact with system 10 to view previously displayed images of surgical steps, selectively view images, instruct the CAOS system 10 to render the anatomical result of a proposed surgical step or procedure, or perform other surgical related functions. For example, the surgeon may view rendered images of the resulting bone structure of different bone resection procedures. In addition, the surgeon may interact with the CAOS system 10 to control various devices of the system 10. For example, the surgeon may interact with the system 10 to control user preferences or settings of a display device, such as a monitor, included in the CAOS system 10. Further, the CAOS system 10 may prompt the surgeon or other user of the system 10 for responses. For example, the CAOS system 10 may prompt the surgeon to inquire if the surgeon has completed the current surgical step, if the surgeon would like to view other images, and the like.

In use, the surgeon interacts with the CAOS system 10 via the microphone 12. That is, the surgeon interacts with the CAOS system 10 by speaking voice commands into the microphone 12. The microphone 12 transmits the voice commands to the voice recognition device 14 via the communication link 16. The voice recognition device 14 interprets the voice commands or otherwise produces an output signal based on the voice commands. Other components of the CAOS system 10, such as a computer, perform surgical related functions based on the output signal. For example, the surgeon can control the CAOS system 10 by speaking the appropriate voice command (e.g. "START", "NEXT IMAGE", "BACK", etc.) to display the next sequential surgical step image, display any selected image, change user preferences, or otherwise perform any interaction with the CAOS system 10 that is typically performed via use of direct interaction devices such as a keyboard, mouse, and touch screen. To do so, the voice recognition device 14 is trained or otherwise programmed to respond to a voice command corresponding to the various functions of the CAOS system 10 which are to be voice controlled. The selection of voice commands may be pre-programmed into the voice recognition device 14 or may be learned or refined over time via use by the surgeon (i.e., the voice recognition device 14 may become more responsive and/or have an increased word recognition accuracy as the surgeon uses the CAOS system 10 over time). In one particular embodiment, the voice commands to which the voice recognition device 14 is programmed to respond is a predetermined selection of voice commands directed specifically to the functionality of the CAOS system 10. By limiting the number of recognized voice commands, the voice recognition device 14 may have an increased voice command recognition accuracy.

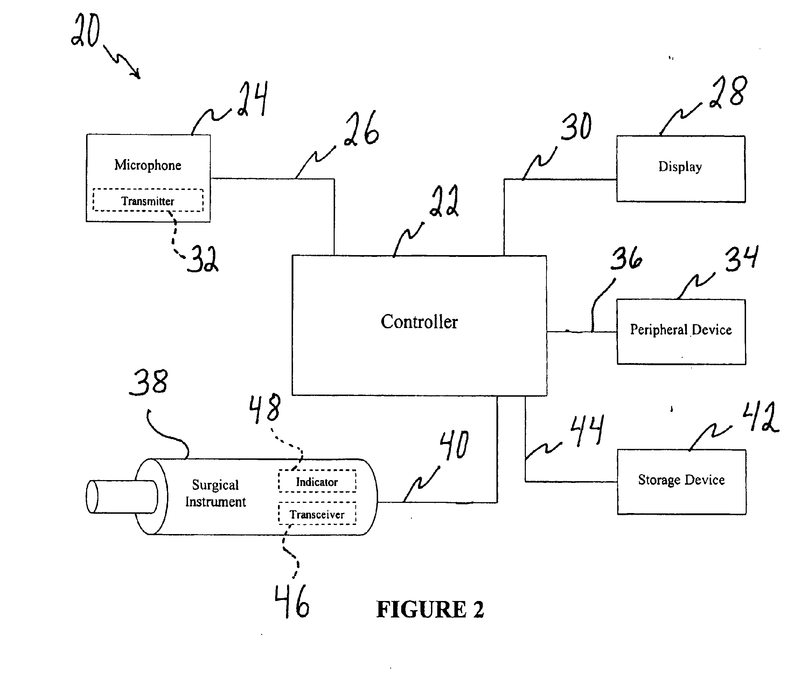

Referring now to FIG. 2, a surgical system 20 for assisting a surgeon in performing an orthopaedic surgical procedure includes a controller 22, a microphone 24 communicatively coupled to the controller 22 via a communication link 26, and a display device 28 electrically coupled to the controller 22 via a communication link 30. The controller 22 may be any type of controller including, but not limited to, a personal computer, a specialized microcontroller device having, for example, an application specific integrated circuit (ASIC), or a CAOS system. The microphone 24 may be similar to the microphone 12 of the system 10 illustrated in FIG. 1. For example, the microphone 24 may be wired or wireless and may be worn by the surgeon, held, or mounted. If the microphone 24 is wireless, the microphone 24 may include a transmitter 32 configured to transmit the voice commands received by the microphone 22 to the controller 24 via the communication link 26. The communication link 26 may be a wired or wireless communication link depending on the type of microphone used. The display device 28 may be any type of display device such as a display monitor. It should be appreciated that a display device 28 need not be dedicated for voice recognition capability, but rather the display device 28 may be one in the same with the display device of the CAOS system.

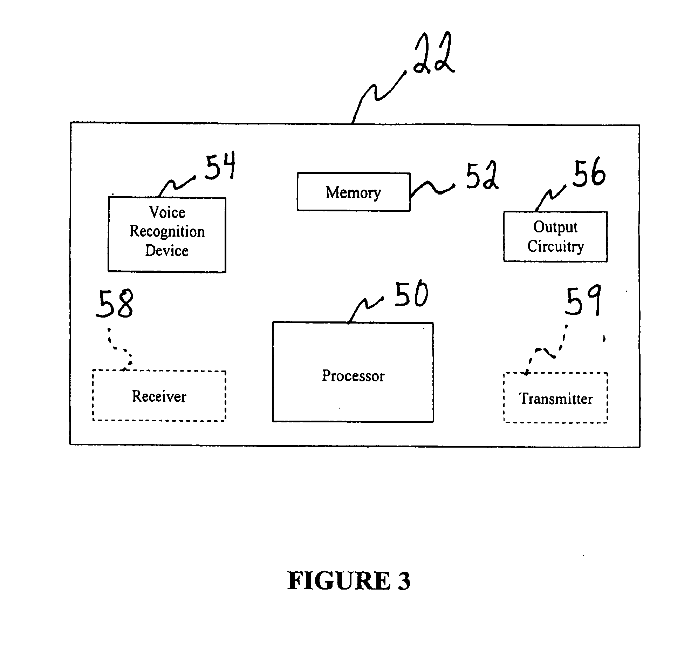

Referring now to FIG. 3, in one embodiment, the controller 22 includes a processor 50, a memory device 52, a voice recognition device 54, and output circuitry 56. The processor 50 may be any type of processor including, but not limited to, a microprocessor, a microcontroller, or an ASIC. Similar to the voice recognition device 14 of the system 10 of FIG. 1, the voice recognition device 54 may be embodied as electrical hardware, software, or a combination thereof. For example, the voice recognition device 14, or portion thereof, may be embodied as a software program stored in the memory device 52 and executed by a processor 50. In one particular embodiment, the voice recognition device 54 can use software that is sold by Scansoft of Peabody, Massachusetts under the trade mark Dragon NaturallySpeaking Medical Software (version 8). The output circuitry 56 includes all such circuitry commonly found in computer system for controlling and interacting with peripheral devices coupled to the computer systems. For example, the output circuitry 56 includes circuitry for controlling the display device 28, controlling other peripheral devices connected to the controller 22, and the like.

In some embodiments, the controller 22 may also include a receiver 58 and a transmitter 59. The receiver 58 may be configured to receive wireless signals. For example, in embodiments in which the microphone 24 is a wireless microphone, the receiver 58 is configured to wirelessly receive the voice commands transmitted from the microphone 24. Once received, the receiver 58 transmits the voice commands to the processor 50 and/or voice recognition device 54 for the interpretation or otherwise processing of the voice commands. The transmitter 59 may be configured to communicate with any number of peripheral devices such as, for example, "smart" medical devices including smart surgical instruments, smart surgical trials, smart surgical implants, and the like. In some embodiments the receiver 58 and transmitter 59 are embodied as one or more transceivers.

Referring back to FIG. 2, the system 20 may also include a number of peripheral devices 34, one or more storage devices 42, and/or any number of surgical instruments 38. The peripheral device 34 may include any type of peripheral devices used in or used to assist in the surgical procedure. For example, the peripheral devices 34 may include lights, surgical table actuation motors, and the like. The peripheral devices 34 are coupled to the controller 22 via a corresponding number of communication links 36. Any number of the communication links 36 may be wired or wireless. The controller 22 may communicate with the peripheral devices 34 using any suitable communication technology an/or protocol including, but not limited to, USB, TCP/IP, Bluetooth, ZigBee, Wi-Fi, Wireless USB, and the like. One communication network that may be used to enable wireless communication between the controller 22, the peripheral devices 34, and other devices is disclosed in the European patent EP-A-1677225.

The storage device 42 may be embodied as any type of storage device such as, for example, a hard drive, memory device, or removable media device such as a compact disk drive, a digital-video-disk drive, or any other storage device. The storage device, amongst other things, maintains the operating and application software associated with the system, including the voice recognition software and its associated files. The storage device 42 may be coupled to the controller 22 via an interconnect 44 such wires or cables. The storage device 42 may be embodied as a discrete device, or may be integrated with, or otherwise a part of, a CAOS system.

The surgical instruments 38 may include any type of surgical instruments used in or to assist with an orthopaedic surgical procedure. For example, the surgical instruments 38 may include endoscopes, surgical burrs, and/or ligament balancers. The surgical instruments 38 are communicatively coupled to the controller 22 via a number of communication links 40. Similar to communication links 36, any number of the communication links 40 may be wired or wireless and may communicate with the controller 22 using any suitable communication technology an/or protocol including, but not limited to, USB, TCP/IP, Bluetooth, ZigBee, Wi-Fi, Wireless USB, and the like. As such, some of the surgical instruments 38 may include a transmitter, a receiver, or a transceiver 46 for this purpose. In addition, some of the surgical instruments 38 may include an indicator 48. The indicator 48 may be any type of indicator capable of drawing attention to the instrument 38. For example, the indicator may be a visual, audible, or tactile indicator such as a vibrator.

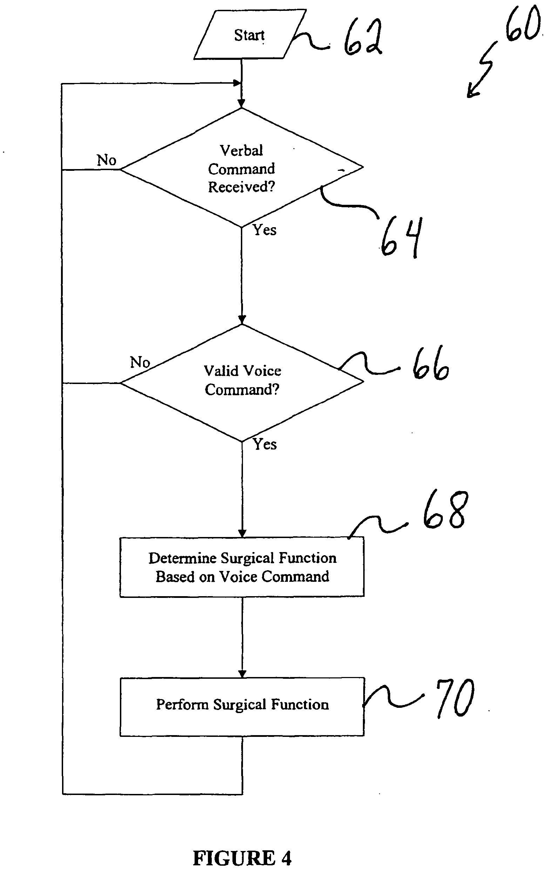

Referring now to FIG. 4, an algorithm 60 for assisting a surgeon in performing an orthopaedic surgical procedure may be executed by the surgical system 20. Although the algorithm 60 will be described in the context of the system 20 of FIGS. 2 and 3, it should be appreciated that the algorithm 60, with or without modification thereto, may also be executed by the surgical system 10 of FIG. 1. The algorithm 60 may be embodied as a software program stored in the memory device 52 and executed by the processor 50 of the controller 22. The algorithm 60 begins with process step 62 in which any variables and/or devices are initialized. In process step 64, the controller 22 determines if a voice command has been received. The controller 22 receives voice commands via the microphone 24. That is, a surgeon interacts with the system 20 by speaking voice commands into the microphone 24. The microphone 24 transmits the voice commands to the controller 22 via the communication link 26. If no voice command has been received, the algorithm 60 loops back to process step 64. In this way, the algorithm 60 continues to check for the receipt of a voice command.

If a voice command has been received by the controller 22, the controller 22 determines if the voice command is a valid or otherwise recognized voice command in process step 66. To do so, the voice recognition device 54 of the controller 22 determines if the received voice command matches or approximately matches one of a selection of recognized voice commands. The voice recognition device 54 may be trained or otherwise programmed to respond to a selection of voice commands corresponding to the various surgical related functions of the system 20. The selection of voice commands may be pre-programmed into the voice recognition device 54 or may be learned or refined over the use of the system 20 by the surgeon. In one particular embodiment, the voice commands to which the voice recognition device 54 is programmed to respond forms a predetermined selection of voice commands specifically directed to the functionality of the system 20.

If the voice command is not a valid voice command, algorithm 60 loops back to process step 64 in which the algorithm 60 continues to monitor for additional voice commands. However, if the voice command is valid, algorithm 60 advances to process step 68 in which the controller 22 determines a surgical related function of the system 20 based on the voice command. Subsequently in process step 70, the controller 22 performs the surgical related function and the algorithm 60 loops back to process step 64 to continue to monitor for additional voice commands. For example, the voice recognition device 54 may produce an output signal based on the recognized voice command and the processor 50 may be configured to perform or initiate the performance of a corresponding surgical related function in response to the output signal (i.e., in response to the voice command). In this way, a surgeon may control the system 20 to perform any one of a number of surgical related functions by speaking a corresponding voice command.

The surgical related functions performed by the system 20 may include any function that assists the surgeon in the performance of the orthopaedic surgical procedure. In one embodiment, the surgical related functions include displaying images of the individual surgical steps which form the orthopaedic surgical procedure being performed. The images may be graphically rendered images or graphically enhanced photographic images. For example, the images may include three dimensional rendered images of the relevant anatomical portions of a patient. The images may be displayed in sequential surgical order or may be randomly accessed and selectively displayed. In addition, the surgical related functions may include rendering the anatomical result of a proposed surgical step or procedure. For example, the surgeon may view rendered images of the resulting bone structure of different bone resection procedures. To display the requested images, the surgeon speaks the corresponding voice command (e.g., "NEXT IMAGE", "PREVIOUS IMAGE", "RENDER PROCEDURE", etc.) into the microphone 24.

The surgical related functions performed by the system 20 may also include control of and communication with the peripheral devices 34. For example, the surgeon may speak a voice command corresponding to an "on" function of a light device coupled to the controller 22 (e.g., "LIGHT ON"). In response, the controller 22 is configured to control or otherwise communicate with the peripheral light device 34 to cause the device 34 to turn on. In addition to "on" and "off' control of the peripheral devices 34, the controller 22 may be configured to send or receive data from the peripheral devices 34 in response to corresponding voice commands (e.g., "GET DATA").

The surgical related functions performed by the controller 22 may further include control of and communication with one or more of the surgical instruments 38. For example, the controller 22 may be configured to identify a specific surgical instrument 38 in response to a corresponding voice command (e.g., "FIND BURR", "IDENTIFY ENDOSCOPE", "WHICH INSTRUMENT IS NEXT", etc.). To do so, the controller 22 determines which instrument 38 is being referred to by the voice command and then communicates with the specific surgical instrument 38 via, for example, transceiver 46 to cause the activation of the indicator 48 of the specific instrument 38. For example, the controller 22 may be configured to remotely activate a visual, audible, or tactile indicator 48 of a surgical burr instrument 38 in response to a corresponding voice command such as "FIND BURR." In addition to identifying requested surgical instruments 38, the controller 22 may also be configured to communicate with one or more of the surgical instruments 38 to receive or send data to the instrument 38 in response to a corresponding voice command. The data received from the surgical instrument may, for example, be subsequently displayed on the display device 28. For example, a surgeon may instruct the controller 22, via a corresponding voice command, such as "GET DATA", to communicate with one of the surgical instruments 38, such as a knee ligament balancer, to retrieve measurement data of the instrument 38 and then display the measurement data on the display device 28 for viewing by the surgeon.

Additionally, the surgical related functions may include surgical note recording. For example, the controller 22 may be configured to begin recording verbal communication from the surgeon after receiving a corresponding voice command to do so (e.g., "BEGIN RECORDING"). The controller 22 may also be instructed to stop recording via a corresponding voice command (e.g., "STOP RECORDING"). The controller 22 may store the verbal communication in storage device 42 for later retrieval and review by the surgeon. The controller 22 may store the verbal communication using any suitable electronic format including, but not limited to, audio formats such as the wave format (.wav) and the MPEG-2 Layer 3 format (.mp3). Moreover, the controller 22 may convert and store the verbal communication in a text format. For example, the controller 22 may convert and store the verbal communication digitally, for example in plain text format, or in rich text format, or in some other text format. In addition, the controller may be configured to communicate with a printer at the request of the user to generate a text hard copy of the recorded verbal communication.

In some embodiments, the surgical related functions may include the creation of an audio-visual record of the surgical procedure. In such case, the controller 22 is configured to record the images of the surgical steps displayed to the surgeon along with any verbal commentary provided by the surgeon during the procedure. For example, the controller 22 may begin recording the images of the surgical procedure and any verbal communication from the surgeon in response to a corresponding verbal command (e.g., "BEGIN AUDIO AND VISUAL RECORDING"). The controller 22 may store the images and the associated commentary from the surgeon in the memory device 52, the storage device 42, or some other media storage device coupled to the controller 22. Additionally, the controller 22 may be configured to determine and store parameters related to the images of the surgical procedure. For example, the controller 22 may determine the length of time a particular image was viewed by the surgeon, the sequence in which the images were viewed, verbal commands received by the controller 22 while displaying of the images, and the like. The controller 22 may store the image parameters along with the images and the verbal commentary. Alternatively, the controller 22 may use the image parameters to determine how to store the images and/or verbal commentary. For example, the controller 22 may store the images of the surgical procedure according to how they were viewed by the surgeon during the procedure rather than sequentially.

After the surgical procedure is completed, the surgeon may review the stored audio-visual record and provide additional dictation, which is then incorporated into the audio-visual record by the controller 22. For example, the surgeon may review the stored audio-visual record and provide additional surgical details for each image. Such details may include, for example, the type of implant used, any variations of the surgical procedure, the types of tools used to perform the surgical procedure, complications encountered during the surgical procedure, amount of tissue or bone removed, and the like. Once the audio-visual record is completed (e.g., any additional dictation is added), the record may be uploaded to a remote database, such as a hospital database, for storage and later retrieval by other medical personnel.

## Claims

1. A system (10, 20) for assisting a surgeon in performing an orthopaedic surgical procedure, the system (10, 20) comprising:
a display device (28),
a microphone (12, 24) configured to transmit voice commands from the surgeon,
and
a controller (22) electrically coupled (30) to the display device (28) and communicatively coupled (26) to the microphone (12, 24), the controller (22) being configured to control the display device (28) to display a number of images of the orthopaedic surgical procedure in response to at least one of the voice commands; **characterized by** the system further comprising
an orthopaedic surgical instrument (38) having a receiver and an indicator (48),
wherein the controller (22) includes a transmitter (59) and is configured to communicate with receiver of the orthopaedic surgical instrument (38) via the transmitter (59) to cause activation of the indicator (48) in response to a voice command which identifies the orthopaedic surgical instrument (38).

2. The system (10, 20) of claim 1, wherein the microphone (12, 24) is a wireless microphone.

3. The system (10, 20) of claim 1, wherein the controller (22) forms a portion of a computer assisted orthopaedic surgery system.

4. The system (10, 20) of claim 1, wherein the controller (22) is configured to be trained to recognize and be responsive to the voice commands.

5. The system (10, 20) of claim 1, wherein the number of images include sequential images of corresponding sequential surgical steps of the orthopaedic surgical procedure, and the controller (22) is configured to control the display device (28) to display one of the sequential images corresponding to a current sequential step of the orthopaedic surgical procedure and to display the next sequential image in response to a voice command indicating the surgeon has completed the current sequential step.

6. The system (10, 20) of claim 5, wherein the controller (22) is configured to control the display device (28) to display a previously displayed sequential image in response to at least one of the voice commands indicating a request to view the previously displayed sequential image.

7. The system (10, 20) of claim 1, wherein the controller (22) is configured to control the display device (28) to modify one of the number of images to graphically render the resulting anatomical effect of a proposed action by the surgeon in response to at least one of the voice commands.

8. The system (10, 20) of claim 1, wherein the indicator (48) includes at least one of a visual indicator and a vibrator.

9. The system (10, 20) of claim 1, in which:
the orthopaedic surgical instrument (38) further includes a transmitter and the controller (22) further includes a receiver (58),
the orthopaedic surgical instrument (38) is configured to transmit data to the controller (22), and
the controller (22) is configured to control the display device (28) to display the data in response to at least one of the voice commands.

## Patentansprüche

1. System (10, 20) zur Unterstützung eines Chirurgen bei der Durchführung eines orthopädischen Eingriffs, wobei das System (10, 20) umfasst:
eine Anzeigeeinrichtung (28),
ein Mikrofon (12, 24), das zum Senden von Sprachbefehlen von dem Chirurgen konfiguriert ist, und
eine Steuereinrichtung (22), die mit der Anzeigeeinrichtung (28) elektrisch gekoppelt und mit dem Mikrofon (12, 24) kommunikativ gekoppelt (26) ist, wobei die Steuereinrichtung (22) zum Steuern der Anzeigeeinrichtung (28) zum Anzeigen einer Anzahl von Bildern des orhopädischen Eingriffs als Reaktion auf mindestens einen der Sprachbefehle konfiguriert ist; **dadurch gekennzeichnet, dass** das System ferner umfasst
ein orthopädisches Operationsinstrument (38) mit einem Empfänger und einer Anzeigeeinrichtung (48), wobei die Steuereinrichtung (22) einen Sender (59) enthält und zum Kommunizieren mit dem Empfänger des orthopädischen Operationsinstruments (38) über den Sender (59) konfiguriert ist, um eine Aktivierung der Anzeigeeinrichtung (48) als Reaktion auf einen Sprachbefehl, der das orthopädische Operationsinstrument (38) identifiziert, zu verursachen.

2. System (10, 20) nach Anspruch 1, **dadurch gekennzeichnet, dass** das Mikrofon (12, 24) ein drahtloses Mikrofon ist.

3. System (10, 20) nach Anspruch 1, **dadurch gekennzeichnet, dass** die Steuereinrichtung (22) einen Abschnitt eines Computer-unterstützten orthopädischen Operationssystems bildet.

4. System (10, 20) nach Anspruch 1, **dadurch gekennzeichnet, dass** die Steuereinrichtung (22) konfiguriert ist, um auf Erkennen der Sprachbefehle und Reagieren darauf trainiert zu werden.

5. System (10, 20) nach Anspruch 1, **dadurch gekennzeichnet, dass** die Anzahl von Bildern sequentielle Bilder von korrespondierenden sequentiellen Operationsschritten des orthopädischen Eingriffs enthält und die Steuereinrichtung (22) zum Steuern der Anzeigeeinrichtung (28) zum Anzeigen von einem der sequentiellen Bilder, die einem aktuellen sequentiellen Schritt des orthopädischen Eingriffs entsprechen, und Anzeigen des nächsten sequentiellen Bildes als Reaktion auf einen Sprachbefehl, der dem Chirurgen anzeigt, dass der aktuelle sequentielle Schritt beendet worden ist, konfiguriert ist.

6. System (10, 20) nach Anspruch 5, **dadurch gekennzeichnet, dass** die Steuereinrichtung (22) zum Steuern der Anzeigeeinrichtung (28) zum Anzeigen eines vorangehend angezeigten sequentiellen Bildes als Reaktion auf mindestens einen der Sprachbefehle, der eine Anfrage zum Betrachten des vorangehend angezeigten sequentiellen Bildes anzeigt, konfiguriert ist.

7. System (10, 20) nach Anspruch 1, **dadurch gekennzeichnet, dass** die Steuereinrichtung (22) zum Steuern der Anzeigeeinrichtung (28) zum Modifizieren von einem der Anzahl von Bildern zum grafischen Rendern des resultierenden anatomischen Effekts einer vorgeschlagenen Aktion durch den Chirurgen als Reaktion auf mindestens einen der Sprachbefehle konfiguriert ist.

8. System (10, 20) nach Anspruch 1, **dadurch gekennzeichnet, dass** die Anzeigeeinrichtung (48) eine optische Anzeigeeinrichtung und/oder eine Vibrationseinrichtung enthält.

9. System (10,20) nach Anspruch 1, in dem:
das orthopädische Operationsinstrument (38) ferner einen Sender enthält und die Steuereinrichtung (22) ferner einen Empfänger (58) enthält,
das orthopädische Operationsinstrument (38) zum Senden von Daten an die Steuereinrichtung (22) konfiguriert ist, und
die Steuereinrichtung (22) zum Steuern der Anzeigeeinrichtung (28) zum Anzeigen der Daten als Reaktion auf mindestens einen der Sprachbefehle konfiguriert ist.

## Revendications

1. Système (10, 20) pour assister un chirurgien dans l'exécution d'une procédure chirurgicale orthopédique, le système (10, 20) comprenait
■ un dispositif d'affichage (28),
■ un microphone (12, 24) configuré pour transmettre des commandes vocales du chirurgien, et
■ un contrôleur (22) couplé électriquement (30) au dispositif d'affichage (28) et couplé de manière communicante (26) au microphone (12, 24), le contrôleur (22) étant configuré pour commander le dispositif d'affichage (28) pour afficher un nombre d'images de la procédure chirurgicale orthopédique en réponse à au moins l'une des commandes vocales ; **caractérisé en ce que** le système comprend en outre :
■ un instrument chirurgical orthopédique (38) comportant un récepteur et un indicateur (48), dans lequel le contrôleur (22) comprend un émetteur (59) et est configuré pour communiquer avec le récepteur de l'instrument chirurgical orthopédique (38) par l'intermédiaire de l'émetteur (59) pour provoquer l'activation de l'indicateur (48) en réponse à une commande vocale qui identifie l'instrument chirurgical orthopédique (38).

2. Système (10, 20) selon la revendication 1, dans lequel le microphone (12, 24) est un microphone sans fil.

3. Système (10, 20) selon la revendication 1, dans lequel le contrôleur (22) forme une partie d'un système de chirurgie orthopédique assisté par ordinateur.

4. Système (10, 20) selon la revendication 1, dans lequel le contrôleur (22) est configuré pour être formé pour reconnaître et réagir aux commandes vocales.

5. Système (10, 20) selon la revendication 1, dans lequel le nombre d'images comprend des images séquentielles d'étapes chirurgicales séquentielles correspondantes de la procédure chirurgicale orthopédique, et le contrôleur (22) est configuré pour commander le dispositif d'affichage (28) pour afficher l'une des images séquentielles correspondant à une étape séquentielle actuelle de la procédure chirurgicale orthopédique et pour afficher l'image séquentielle suivante en réponse à une commande vocale indiquant que le chirurgien a achevé l'étape séquentielle actuelle.

6. Système (10, 20) selon la revendication 5, dans lequel le contrôleur (22) est configuré pour commander le dispositif d'affichage (28) pour afficher une image séquentielle affichée précédemment en réponse à au moins l'une des commandes vocales indiquant une demande pour voir l'image séquentielle affichée précédemment.

7. Système (10, 20) selon la revendication 1, dans lequel le contrôleur (22) est configuré pour commander le dispositif d'affichage (28) pour modifier l'une du nombre d'images pour présenter graphiquement l'effet anatomique résultant d'une action proposée par le chirurgien en réponse à au moins l'une des commandes vocales.

8. Système (10, 20) selon la revendication 1, dans lequel l'indicateur (48) comprend au moins l'un d'un indicateur visuel et d'un vibreur.

9. Système (10, 20) selon la revendication 1, dans lequel :
■ l'instrument chirurgical orthopédique (38) comprend en outre un émetteur et le contrôleur (22) comprend en outre un récepteur (58),
■ l'instrument chirurgical orthopédique (38) est configuré pour transmettre des données au contrôleur (22), et
■ le contrôleur (22) est configuré pour commander le dispositif d'affichage (28) pour afficher les données en réponse à au moins l'une des commandes vocales.
